# EUROPEAN PATENT SPECIFICATION

(11) **EP 2 790 537 B1**
(45) Date of publication and mention of the grant of the patent: **11.04.2018**
(21) Application number: 12859021.3
(22) Date of filing: 09.12.2012
(51) Int. Cl.: A24F 47/00

(54) **ELECTRONIC CIGARETTE CHARGING SYSTEM COMPRISING AN ELECTRONIC CIGARETTE WITH A MAGNETIC ELECTRICAL CONTACT IN FORM OF AN OUTER RING**
LADESYSTEM FÜR EINE ELEKTRONISCHE ZIGARETTE UMFASSEND EINE ELEKTRONISCHE ZIGARETTE MIT EINEM MAGNETISCHEN ELEKTRISCHEN KONTAKT IN FORM EINES ÄUSSEREN RINGS
SYSTÈME DE CHARGEMENT POUR UNE CIGARETTE ÉLECTRONIQUE COMPRENANT UNE CIGARETTE ÉLECTRONIQUE AVEC UN CONTACT ÉLECTRIQUE MAGNÉTIQUE EN FORM D'UN ANNEAU EXTÉRIEUR

(30) Priority: 18.12.2011 US 201161577024 P
(43) Date of publication of application: 22.10.2014
(73) Proprietor: SIS Resources Ltd., 99000 Beit Shemesh (IL)
(72) Inventor: WEIGENSBERG, Aaron Arye, 71720 Modiin (IL); GAVRIELOV, Shmuel, 93877 Jerusalem (IL); CAPUANO, Sammy, 99095 Beit Shemesh (IL)
(74) Representative: Docherty, Andrew John
(86) International application number: PCT/IB2012/057108
(87) International publication number: WO 2013/093695

(56) References cited:
- EP-A1- 2 022 349
- EP-A1- 2 100 525
- CN-A- 101 869 356
- CN-U- 201 491 721
- GB-A- 2 445 598
- JP-A- 2011 087 569
- US-A- 5 322 075
- US-A1- 2005 134 215
- US-A1- 2010 307 518
- US-A1- 2010 313 901
- US-A1- 2011 304 282

## Description

### FIELD OF THE INVENTION

This invention relates to tobacco product avoidance. More particularly, this invention relates to an electronic cigarette charging system including a rechargeable electronic cigarette for limiting exposure to tobacco fumes and a cradle connectable to a battery charger, the cradle comprising a receiving element to receive an end of the electronic cigarette.

### BACKGROUND OF THE INVENTION

Tobacco-containing smoking devices are known. For example, U.S. Patent No. 7,726,320 proposes a cigarette incorporated within an electrically powered aerosol generating device that acts as a holder for that cigarette. The smoking device possesses at least one form of tobacco. The smoking device also possesses a mouth-end piece that is used by the smoker to inhale components of tobacco that are generated by the action of heat upon components of the cigarette. A representative smoking device possesses an outer housing incorporating a source of electrical power (e.g., a battery), a sensing mechanism for powering the device at least during periods of draw, and a heating device (e.g., at least one electrical resistance heating element) for forming a thermally generated aerosol that incorporates components of tobacco. During use, the cigarette is positioned within the device, and after use, the used cigarette is removed from the device and replaced with another cigarette. The document CN 101 869 356 A discloses a rechargeable electronic cigarette with outer and inner contacts and means for charging the electronic cigarette. European patent document EP 111736 065 proposes an electronic cigarette containing nicotine without tar, which includes a shell and a suction nozzle. On the exterior wall of the shell, there is an air orifice, while an electronic circuit board, a constant pressure cavity, a sensor, a gas liquid separator, an atomizer, and a supplying bottle are orderly located in the interior of the shell, wherein the electronic circuit board consists of an electronic switching circuit and a high-frequency generator. At one side of the sensor there is an air duct. A negative pressure cavity is located in the sensor. The atomizer connects with the supplying bottle, and there is an atomizing cavity located in the atomizer.

Devices of this sort are referred to herein for convenience as "electronic cigarettes". Conventionally the atomizer of an electronic cigarette includes a heating element, which is typically a wire having high electrical resistance. Rechargeable batteries housed within the electronic cigarette are generally used to power the atomizer. Thus, during use it becomes necessary to recharge the batteries from time to time. Accomplishing this may require some disassembly of the electronic cigarette in order to connect an external charging device. It is impractical to smoke using the electronic cigarette while charging the device without actually replacing the battery. To do so, it would be necessary to repeatedly disconnect the charger, reassemble the electronic cigarette, puff, and then reverse the procedure to continue the charging process.

### SUMMARY OF THE INVENTION

The present invention seeks to provide an improved electronic cigarette, which is connectable to a battery charger without disassembly of the cigarette. An electronic cigarette charging system is provided, the system including a rechargeable electronic cigarette having first and second electrical contacts and a cradle that is connectable to a battery charger and includes a receiving element adapted to receive the end of the electronic cigarette. The receiving element has electrically conductive first and second contacts, which have first and second contact surfaces for contacting the first and second electrical contacts of the electronic cigarette, respectively to thereby establish an electrical connection with the battery charger.

According to a further aspect of the system, the first and second electrical contacts of the electronic cigarette are magnetically attractive to the first and second contact surfaces of the receiving element, respectively.

According to yet another aspect of the system, at least one of the first and second contact surfaces of the receiving element is a magnet.

According to still another aspect of the system, at least one of the first and second contacts of the electronic cigarette is a magnet.

According to an aspect of the system, the end of the electronic cigarette includes an adaptor, which has an orifice formed therethrough and an inset for receiving a conductive flange. The orifice is disposed such that an extension of the flange is placed in contact with one of the first and second contacts of the receiving element when the electronic cigarette is in the cradle. The adaptor includes at least one side groove for receiving a contact pin to contact another of the first and second contacts of the receiving element.

According to a further aspect of the system, the adaptor has vents formed therein to permit ambient air to enter an interior of the electronic cigarette.

According to still another aspect of the system, the end of the electronic cigarette includes an adaptor having a sensor unit housed in an interior chamber thereof, and has a perforation extending from the interior chamber to an exterior of the electronic cigarette to place the interior chamber in fluid communication with an ambient atmosphere.

According to still another aspect of the system, the end of the electronic cigarette includes an adaptor, whereby the adaptor has a cap. A lip and a conductive flange on the cap are adapted for contacting one of the first and second contact surfaces of the receiving element. A conductive coaxial member on the adaptor has an extension, and an insert attached to the lip, wherein the insert has an orifice formed therethrough for receiving the extension of the coaxial member. The orifice is disposed such that the extension of the coaxial member is placed in contact with another of the first and second contacts of the receiving element when the electronic cigarette is in the cradle.

According to one aspect of the system, the adaptor has vents formed therein to permit ambient air to enter an interior of the electronic cigarette.

According to an additional aspect of the system, the insert has a translucency and diffusing property sufficient to prevent recognition of structures and shadows within the electronic cigarette when the insert is illuminated from by a light source disposed within the electronic cigarette. An electronic cigarette charging system is further provided. the system including an electronic cigarette having a first set of electrical contacts, an atomizer and a rechargeable battery for powering the atomizer. An electrode assembly connectable to a power source has a second set of electrical contacts that are arranged to mate with the first set of electrical contacts, whereupon the electronic cigarette receives power via the electrode assembly for recharging the battery while remaining operational for use by a smoker. Electrical circuitry is connected to the first set of electrical contacts, the electrical circuitry including a first circuit for supplying battery power to the atomizer and a second circuit for providing power from a battery charger to the battery for recharging thereof. The first circuit is cooperative with the second circuit to disable power flow via the electrode assembly to the battery when the atomizer is activated and to resume the power flow when the atomizer ceases to be activated.

According to one aspect of the system, the second set of electrical contacts includes respective ferromagnetic disks that are bonded to lead wires that are connectable to the battery charger, and magnets held by magnetic attraction in contact with the disks.

According to an aspect of the system, the first set of electrical contacts are disposed at an end of the electronic cigarette.

According to a further aspect of the system, the first set of electrical contacts are disposed at a side portal of the electronic cigarette. According to a further aspect of the system, the system includes a cradle receiving the electronic cigarette and has the second set of electrical contacts disposed therein.

An additional aspect of the system includes a universal serial bus adaptor linked to the second set of electrical contacts for connection to the power source.

According to yet another aspect of the system, the electrical circuitry includes a pressure sensor, a transistor coupled to the battery and the first set of electrical contacts, a microprocessor, and electronic logic, wherein the microprocessor is responsive to the sensor and the electronic logic to regulate the transistor to enable and disable power flow to the battery via the first set of electrical contacts. An electronic cigarette is provided, the cigarette including an atomizer, a rechargeable battery for powering the atomizer, and an adaptor that has a first set of electrical contacts connectable to a battery charger. The adaptor is disposed at an end of the electronic cigarette and includes an outer metallic band having an extension that is exposed to an interior space of the electronic cigarette, an inner metallic plug exposed to the interior space of the electronic cigarette, and an electrically nonconductive intermediate member separating the band from the plug and having a channel formed therein that extends from an exterior of the electronic cigarette to the interior space thereof allowing ambient air to enter the interior space.

According to one aspect of the electronic cigarette, the intermediate member has a translucency and diffusing property sufficient to prevent recognition of structures and shadows within to the electronic cigarette when the adaptor is illuminated from within the electronic cigarette. An electronic cigarette is further provided, the cigarette including a first set of electrical contacts, an atomizer and a rechargeable battery for powering the atomizer, wherein the first set of electrical contacts is connectable to an electrode assembly that has a second set of electrical contacts, whereupon the electronic cigarette receives power via the electrode assembly for recharging the battery while being smoked by a smoker, and electrical circuitry connected to the first set of electrical contacts. The electrical circuitry includes a first circuit for supplying battery power to the atomizer and a second circuit for providing power from a battery charger to the battery for recharging thereof.

According to still another aspect of the electronic cigarette, the first set of electrical contacts are disposed at an end of the electronic cigarette.

According to yet another aspect of the electronic cigarette, the first set of electrical contacts are disposed at a side portal of the electronic cigarette.

According to a further aspect of the electronic cigarette, the electrical circuitry includes a pressure sensor, a transistor coupled to the battery and the first set of electrical contacts, a microprocessor, and electronic logic, wherein the microprocessor is responsive to the sensor and the electronic logic to regulate the transistor to enable and disable power flow to the battery via the first set of electrical contacts. An electronic cigarette is further provided, the cigarette including an atomizer, a battery for powering the atomizer, and a tip adaptor disposed at an end of the electronic cigarette. The tip adaptor has a sensor unit housed in an interior chamber thereof, and has a perforation extending from the interior chamber to an exterior of the electronic cigarette to place the interior chamber in fluid communication with ambient atmosphere.

According to an additional aspect of the electronic cigarette, the electrical circuitry is configured for selective operation in a first mode, wherein power flow to the battery is disabled when the atomizer is activated by a user drawing on the electronic cigarette and is enabled when the atomizer is not activated and in a second mode, wherein power flow to the battery is enabled when the atomizer is activated by the user drawing on the electronic cigarette and when the atomizer is not activated.

### BRIEF DESCRIPTION OF THE DRAWINGS

For a better understanding of the present invention, reference is made to the detailed description of electronic cigarettes and systems, by way of example, which is to be read in conjunction with the following drawings, wherein like elements are given like reference numerals, and wherein:
Fig. 1 is a semi-schematic exploded view of an electronic cigarette;
Fig. 2 is an elevation of a tip charge adaptor, shown in slight perspective;
Fig. 3 is another perspective view of the adaptor shown in Fig. 2;
Fig. 4 is a cutaway view of the adaptor shown in Fig. 2 shown in side elevation;
Fig. 5 is a sectional view of the adaptor shown in Fig. 2;
Fig. 6 is a cutaway view of the adaptor shown in Fig. 2;
Fig. 7 is an elevation of a cradle for connection to a battery charging device;
Fig. 8 is an elevation of shows the cradle shown in Fig. 7 with an electrical contact subassembly;
Fig. 9 is a side elevation of the cradle shown in Fig. 7;
Fig. 10 is a top view of the cradle shown in Fig. 7;
Fig. 11 is an elevation illustrating magnets of the cradle shown in Fig. 7;
Fig. 12 is an exploded view of an adaptor shown in slight perspective in the smoking device of Fig. 1;
Fig. 13 is a schematic diagram of electrical circuitry in an electronic cigarette adapted to a battery charger;
Fig. 14 is a schematic diagram of electrical circuitry in an electronic cigarette adapted to a battery charger;
Fig. 15 is a schematic diagram of electrical circuitry in an electronic cigarette adapted to a battery charger;
Fig. 16 is a side elevation of a cradle for connection to a battery charging device;
Fig. 17 is a top view of the cradle shown in Fig. 16;
Fig. 18 is a sectional view through the cradle shown in Fig. 16;
Fig. 19 is a partially cut-away view of an electronic cigarette charging system;
Fig. 20, Fig. 21 and Fig. 22 are, respectively, an elevation and two cutaway views of a tip of an electronic cigarette that is constructed and operative to engage a battery charger;
Fig. 23 is a cut-away view of a portion of an electronic cigarette having a LED cap;
Fig. 24 is a partial sectional view of the electronic cigarette as shown in Fig. 23; and
Fig. 25 is a block diagram of electrical circuitry for charging an electronic cigarette.

### DETAILED DESCRIPTION

In the following description, numerous specific details are set forth in order to provide a thorough understanding of the various principles of the present invention. It will be apparent to one skilled in the art, however, that not all these details are necessarily always needed. In this instance, well-known circuits, control logic, and the details of computer program instructions for conventional algorithms and processes have not been shown in detail in order not to obscure the general concepts unnecessarily.

### Definitions.

The terms "link", "linked", "couple" and "couples" are intended to mean either an indirect or direct connection. Thus, if a first device couples to a second device, that connection may be through a direct connection, or through an indirect connection via other devices and connections.

### Device 1.

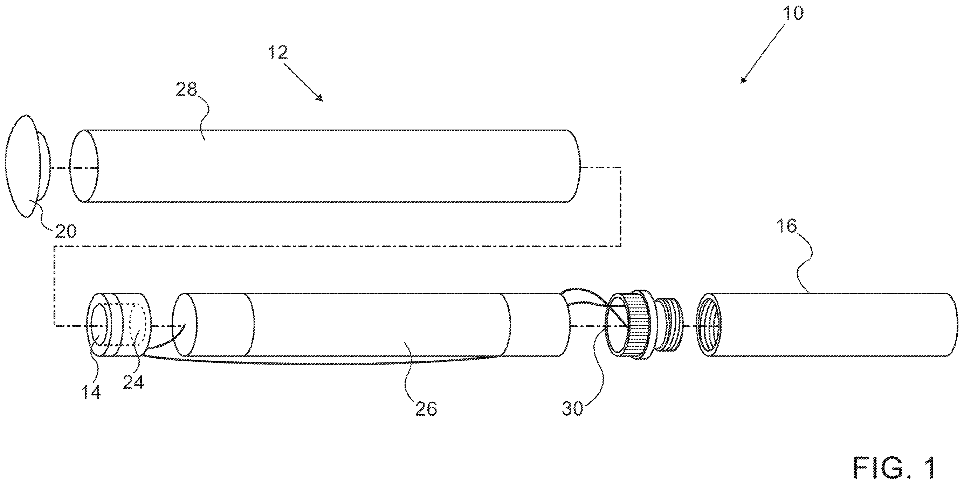

Turning now to the drawings, reference is initially made to Fig. 1, which is a semi-schematic exploded view of a smoking device 10. Device 10 has a barrel comprising a battery section 12. The battery section 12 may include power control circuitry 14, which is typically encased as a unit with a vacuum sensor 24, and which may be enclosed in a plastic holder. The device 10 includes a cartridge section 16, including an atomizer having a high resistance electrical wire, which heats a liquid or gel when the atomizer is powered. The liquid is typically a mixture of nicotine, propylene glycol, vegetable glycerine, and flavorings. The components of the section 16 are integral. In such case, the section 16 is known as a cartomizer. In some cases the power control circuitry 14 may be disposed in the section 16 rather than the battery section 12 as shown in Fig. 1.

In one mode of operation the device 10 is capped by a tip 20. When smoke enters the device 10, an internal aperture (not shown) typically constricts the flow, thereby creating a pressure differential, which is sensed by a vacuum sensor 24 of known type. The electronic power control circuitry 14 linked to the sensor 24 activates the atomizer in the section 16, which is powered, typically by a lithium ion battery 26 that is housed in a barrel 28 of the battery section 12.

An adaptor 30 connects the section 16 and the battery section 12, and may comprise a threaded connector. The power control circuitry may disable the atomizer in the section 16 when the proportion of tobacco fumes exceeds a predefined threshold. Power output to atomizer in section 16 may also be disabled when the battery 26 is being actively charged. Additionally or alternatively all functions of the power control circuitry 14 may be disabled when a battery charger (not shown in Fig. 1) is in operation, as described below.

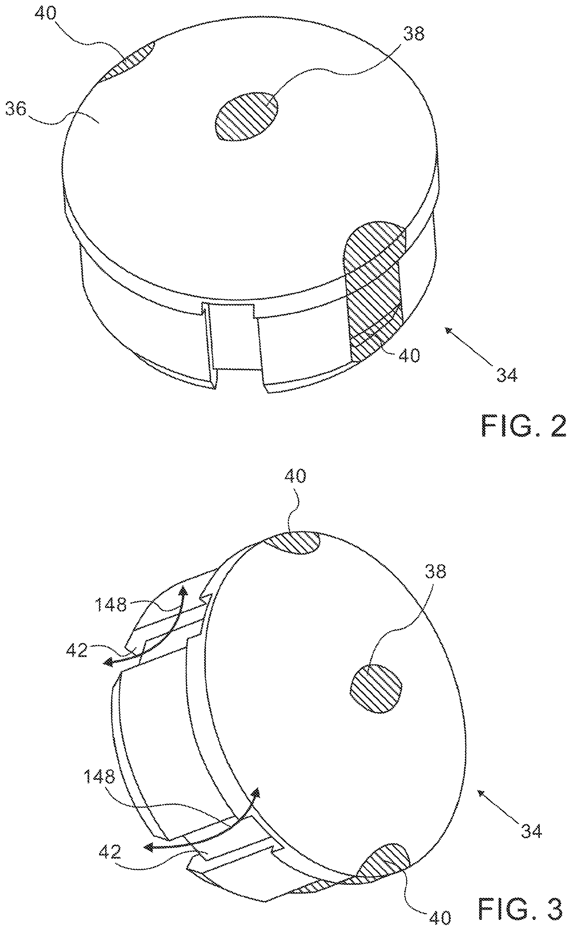

Reference is now made to Fig. 2, which is an elevation of a tip charge adaptor 34, shown in slight perspective. This assembly is compact, comprising a tip section 36 that replaces the tip 20 of the standard device 10 shown in Fig. 1. In addition the adaptor 34 houses the sensor 24 (Fig. 1). A flange 38 is inset in the tip section 36 and provides a contact mechanism with a battery charging device (not shown). The flange 38 is preferably coated with nickel or gold to minimize corrosion and enhance electrical conductivity with the battery charging device. Side openings 40 receive contact pins, which provide a second contact mechanism with the same or a different battery charging device.

Reference is now made to Fig. 3, which is another perspective view of the adaptor 34, illustrating recesses 42. Recesses 42 are side grooves that allow air flow to and from the region below the sensor 24 (Fig. 1), e.g., the battery section 12 (Fig. 1), as indicated by arrows 148. They do not penetrate through the wall of the adaptor 34.

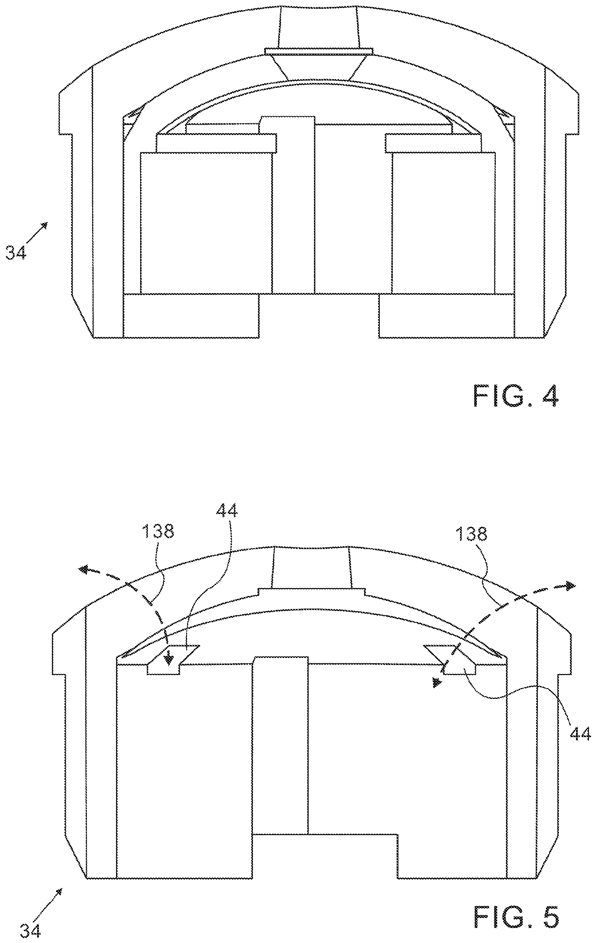

Reference is now made to Fig. 4, which is a cutaway view of the adaptor 34 (Fig. 2) shown in side elevation.

Reference is now made to Fig. 5, which is a sectional view of the adaptor 34 (Fig. 2), showing air vents 44, which penetrate the wall of the adaptor 34, allowing air communication between the exterior and the interior of the adaptor 34, as indicated by arrows 138. The air communication ensures that the top part of sensor 24 (Fig. 1) is exposed to ambient pressure, which is necessary for its proper operation.

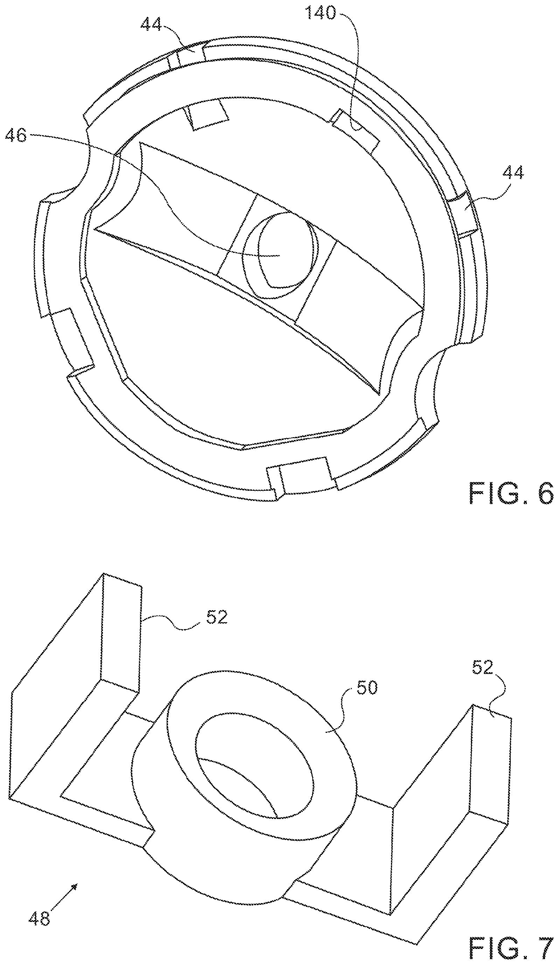

Reference is now made to Fig. 6, which is a cutaway view of the adaptor 34 (Fig. 2) shown as a bottom elevation. An orifice 46 receives the flange 38 (Fig. 2). The air vents 44 are visible on this view. A rectangular groove 140 receives a diode, which is described below in the discussion of the electrical circuitry adapted to a battery charger.

Reference is now made to Fig. 7, which is an elevation of a cradle 48, which is adapted to contain and connect to a battery charging device (not shown) and to receive the adaptor 34 in a concave receiving element 50. Stability is provided by support members 52. The concavity of the element 50 corresponds to a convexity of the adaptor 34. The matching curvatures facilitate a firm electromechanical mating between the cradle 48 and the adaptor 34. Alternatively, firm contact may be achieved by reversing the convexity and matching concavity such that the adaptor 34 presents a concave contacting surface and the cradle 48 has a convex receiving surface. Further alternatively, the element 50 and the adaptor 34 could have flat contacting surfaces.

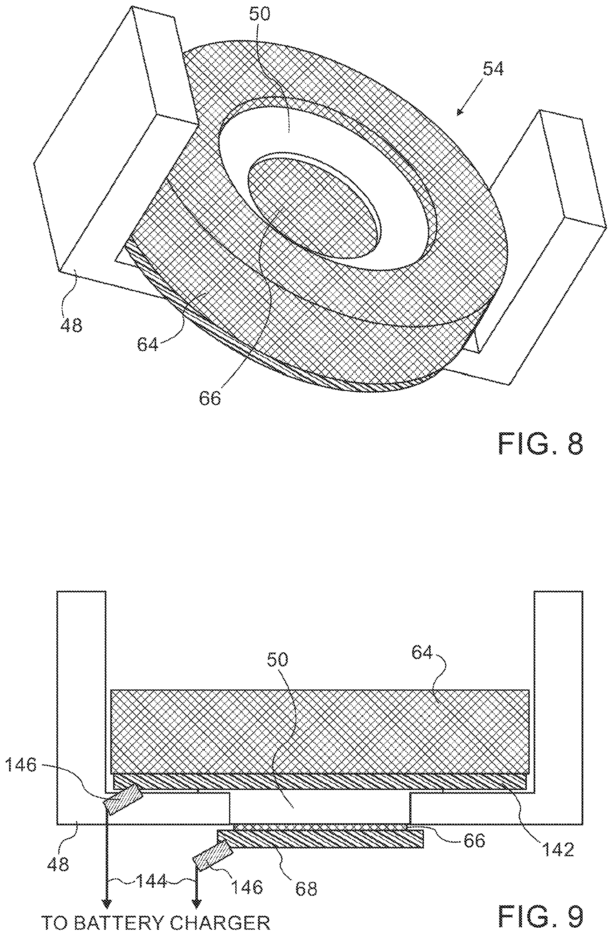

Reference is now made to Fig. 8, which shows the cradle 48 with an electrical contact subassembly 54, and exposes magnetic contact surfaces of magnets 64, 66.

Reference is now made to Fig. 9, which is a partially schematic side elevation of the cradle 48, illustrating magnets 64, 66. The magnet 66 is a cylinder having an exposed contact face as shown on Fig. 9. The magnet 64 is a toroid, which, as shown in Fig. 9, similarly has an exposed contact face. The aperture of the magnet 64 encloses the magnet 66 and the element 50, such that the magnets 64, 66 are spaced apart by the element 50, as can be appreciated by reference to Fig. 7 together with Fig. 9. The magnets 64, 66, form contact points with the battery charging device (not shown). In different cases, one of the magnets 64, 66 may be omitted. In such cases one magnet contact is sufficient to magnetically attract adaptor 34. Disk 68 and ring 142 are formed of a paramagnetic material so as to be held in firm contact with the magnets 64, 66. Electrical leads 144 are soldered to tabs 146, which project from the disk 68 and the ring 142 to create electrical contact between a battery charging device (not shown) and the magnets 64, 66. Use of the disk 68 and the ring 142 and the tabs 146 in this manner avoids soldering the leads 144 to the permanent magnets directly, which would likely damage their magnetism. Alternatively, tabs 146 could be eliminated and electrical leads 144 soldered or spot welded directly to disk 68 and the ring 142.

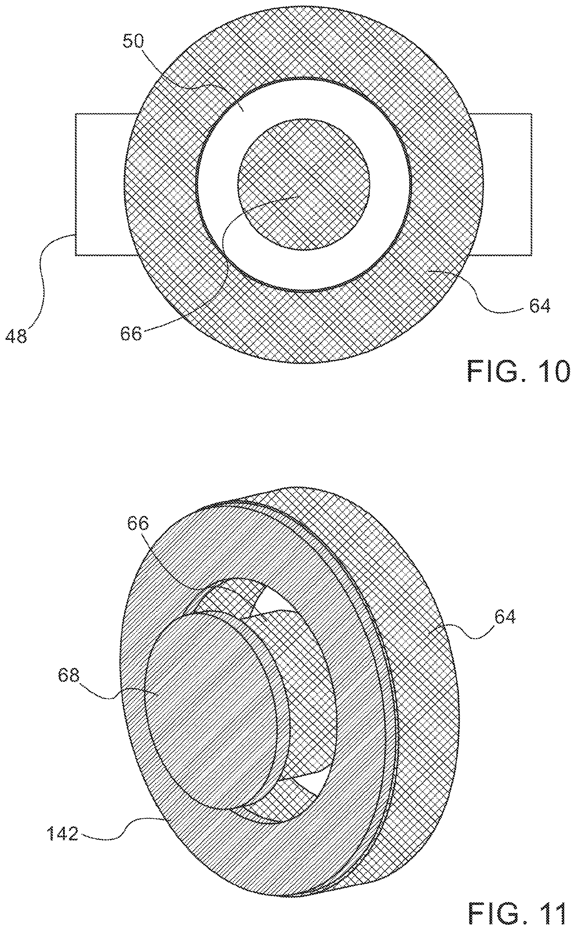

Fig. 10 is a top view of the cradle 48 showing the configuration of the magnets 64, 66 and their relationship to the element 50.

Fig. 11 is an elevation in perspective illustrating the magnets 64, 66 disengaged from the cradle 48 (Fig. 2) with the disk 68 and the ring 142 attached. Alternatively, disk 68 and the ring 142 may be placed above magnet 66 and magnet 64, respectively, wherein the magnets provide attraction to adaptor 34 from below and via the paramagnetic material of disk 68 and ring 142.

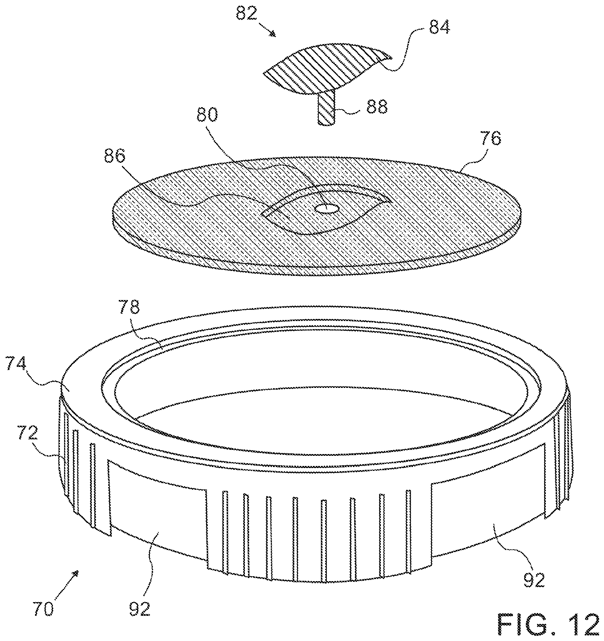

Reference is now made to Fig. 12, which is an exploded composite view of a tip charge adaptor 70, shown in slight perspective. The adaptor 70 may be press-fitted into the end of the device 10 (Fig. 1). Alternatively, a cap 72 on the adaptor 70 may be threaded. The cap 72 has a flange 74 that forms a first electrical contact with a battery charging device (not shown). A plastic insert 76 attaches to a lip 78 formed on the cap 72 and has an aperture 80 bored therethrough to accommodate a metallic coaxial contact 82 having a flat member 84 that may be inset in a corresponding depression 86 of the insert 76. The member 84 functions as a second electrical contact with the battery charging device (not shown). The contact 82 has an extended member 88 that passes through the aperture 80 and attaches to a conductor leading toward a battery being charged. Alternatively, the coaxial contact 82 may be realized in other configurations, e.g., a coaxial ring, and may appear in profile as a domed or pointed tip contact. The member 88 could have many configurations in cross section, for example, tubular, square or rectangular, and could be hollow or solid. Several recesses 92 are formed externally along the circumference of the cap 72 for permitting air flow from outside and into the battery section 12 (Fig. 1) during normal smoking operation.

Preferably, the member 84 and the flange 74 are coated with nickel or gold to minimize corrosion and enhance electrical conductivity with the battery charging device. Contacts on the battery charging device are typically also coated with nickel or gold, and configured to mate with the contacts of the adaptor 70. Details of the battery charger are presented below.

In order that the device 10 (Fig. 1) simulate a lighted cigarette being smoked, the insert 76 is manufactured of a plastic having a translucency and diffusing property so that light from a light emitting diode (LED) (not shown) within the device diffuses at the tip, i.e., throughout the insert 76, so as to prevent recognition of internal structures and shadows by an observer. This effect may be enhanced by including tiny crystals, e.g., glass crystals, within the plastic.

During charging, it is desirable to disable the atomizer or otherwise eliminate it from the charging circuit. Were this not done, the wire (not shown) in the atomizer would constitute a relatively low resistance load on the charger, and would defeat the charging function.

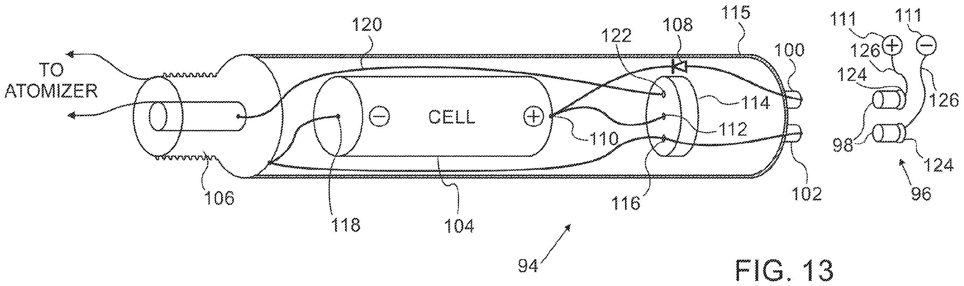

Reference is now made to Fig. 13, which is a schematic diagram of electrical circuitry in an electronic cigarette 94 adapted to a battery charger. A battery charger electrode assembly 96 is provided with magnets 98, which are attracted to external contacts 100, 102. As the contacts 100, 102 incorporate a ferromagnetic material, they are urged into firm electrical contact with the magnets 98 by magnetic attraction. Positive and negative terminals 111 conduct the output of a conventional battery charger (not shown).

In this case, when charging a rechargeable battery 104, it is not necessary to disassemble the cigarette 94, e.g., by unscrewing a threaded metallic connector 106. The cigarette 94 remains intact before, during, and after the charging operation. As will be seen from the following description, the circuit arrangement comprises a first circuit supplying the atomizer of the cigarette 94 and a second charging circuit extending from the contacts 100, 102 to the battery 104.

A diode 108 is interposed in the second circuit between contact 100 and positive terminal 110 of battery 104. The diode 108 permits the battery 104 to be charged, but prevents outflow of electricity to external contacts 100, 102 if the contacts 100, 102 are inadvertently connected so as to cause a short circuit. The positive terminal 110 is also connected to a contact 112 of a pressure sensor chip assembly 114 disposed at or near the tip of the cigarette 94. Another external contact, contact 102, is connected in common to ground point 116 of the sensor chip assembly 114, to negative terminal 118 of the battery 104, and to the connector 106. The connector 106 is typically a threaded connector joining the battery section with the atomizer section of the cigarette 94, as described above with reference to Fig. 1.

A wire 120 connects the atomizer with the sensor chip assembly 114 at a contact point 122. While cigarette 94 is connected to a battery charger, e.g., a USB charger, via assembly 96, if a user draws on the cigarette 94, he causes the atomizer to be actuated in normal smoking operation. As the atomizer is activated, power flow from the battery charger to the battery both charges the battery and powers the atomizer if the battery is not sufficiently charged.

The assembly 96 comprises the magnets 98, which are held by magnetic attraction against ferromagnetic disks 124, to which are bonded (e.g., soldered) wire leads 126 originating from the battery charger (not shown). This construction avoids the difficult operation of soldering the wire leads 126 directly onto the magnets 98, which would likely impair the magnets 98.

### Device 2

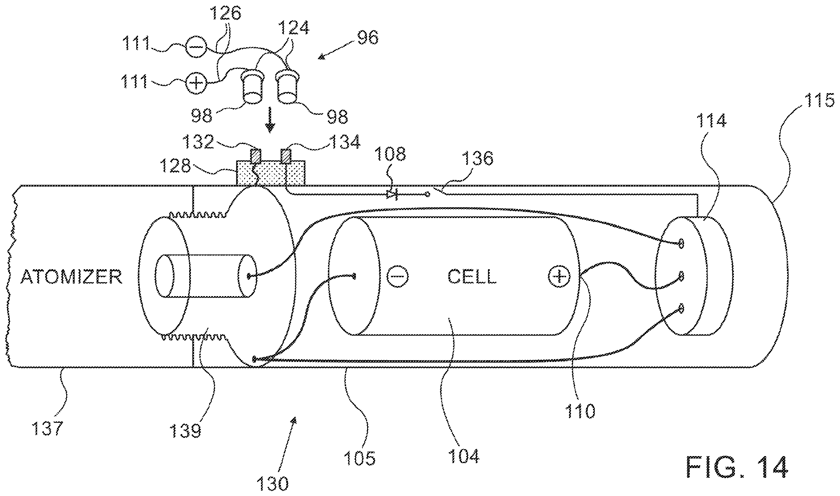

Reference is now made to Fig. 14, which is a schematic diagram of electrical circuitry in an electronic cigarette 130 adapted to a battery charger. With this device it is possible to connect the external charger while powering the atomizer. The battery charger assembly 96 is now connects to a side port 128 of an electronic cigarette 130 having external contacts 132, 134, which connect in a circuit including the diode 108 and the positive terminal 110 of the battery 104 within battery compartment 105, as in the previous case. When the sensor chip assembly 114 is actuated as the user smokes, it actuates a switch, shown representatively as relay 136, thereby breaking the charger circuit so that the charger is no longer operative. Of course, other types of switches, such as a transistor or field effect transistor could be substituted for relay 136. The sensor chip assembly 114 is typically disposed within or near cigarette tip 115. The battery 104 then feeds power to atomizer 137. The atomizer 137 and the battery compartment 105 may be joined by a threaded connector 139. Once the user is no longer inhaling, the sensor chip assembly 114 operates the relay 136 to again make the circuit and enable charging to continue.

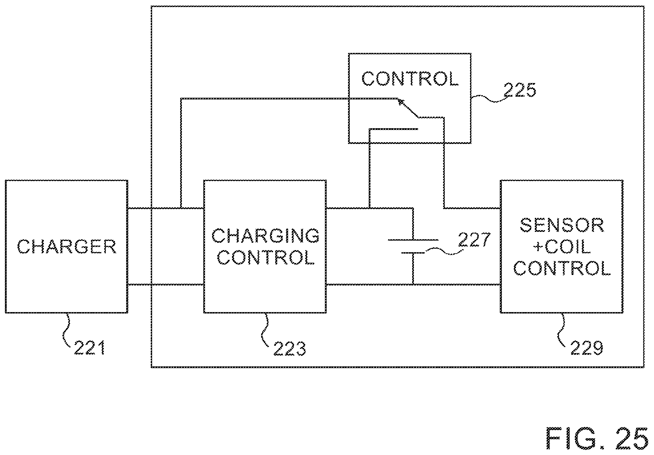

Reference is now made to Fig. 25, which is a block diagram of electrical circuitry for charging an electronic cigarette. Charging control circuitry 223 is provided for charging control and control switch 225 is provided for smoking control. A battery charger 221 can be connected to and disconnected from charging control circuitry 223. The charging control circuitry 223 is linked to the control switch 225, a rechargeable battery 227, and a sensor unit having control circuitry 229. When the battery charger 221 is connected, the control switch 225 enables and disables the charging control circuitry 223 from charging the battery 227 as commanded by control signals from the circuitry 229. The circuitry 229 is actuated to disable the charging control circuitry 223 when a pressure sensor (not shown) detects a pressure difference from the ambient atmosphere, indicating that the user of the electronic cigarette is inhaling. When the sensor fails to detect a pressure difference, the control switch 225 is commanded to enable the charging control circuitry 223, allowing the battery 227 to continue charging.

Specifically, when the control switch 225 detects that the battery charger 221 is connected to the system, it automatically connects the circuitry 229 (sensor and atomizer coil control) to operate directly from the battery charger 221. The battery charger 221 performs two functions, according to the state of the control switch 225: (1) charging the battery; and (2) supplying power to the cigarette for smoking. The only limitation is that the battery charger 221 must have sufficient output capacity to support charging and smoking at the same time.

By suitably configuring the control switch 225, the electrical circuitry may selectively operate in either a first mode, wherein power flow to the battery is disabled when the atomizer is activated by a user drawing on the electronic cigarette and is enabled when the atomizer is not activated or may operate in a second mode, wherein power flow to the battery is enabled when the atomizer is activated by the user drawing on the electronic cigarette and when the atomizer is not activated. In the first mode battery charging is disabled while a puff is being taken on the electronic cigarette. In the second mode battery charging continues concurrently with puffing on the electronic cigarette.

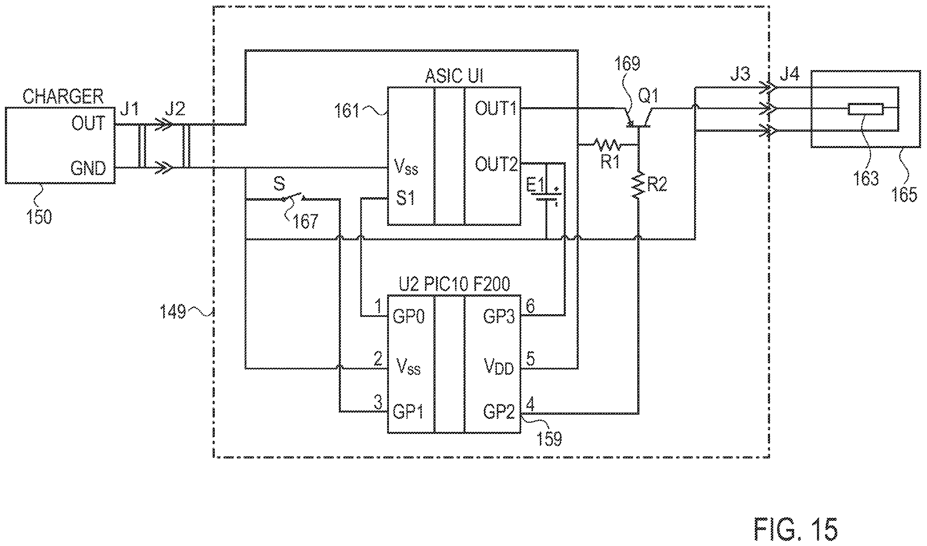

Reference is now made to Fig. 15, which is a detailed schematic diagram of electrical circuitry 149 of an electronic cigarette adapted to a battery charger 150. The circuitry 149 allows concurrent smoking and charging. The circuitry shown in Fig. 14 will only charge the battery - between puffs, i.e., when the atomizer is not actuated.

The following describes the operation of the circuit shown in Fig. 15:

### Scenario A: The charger 150 is not connected.

Microcontroller 159 and application-specific integrated circuit (ASIC 161) are constantly connected to battery cell 163 in cigarette 165. In this state, the output 4-GP2 of microcontroller 159 is set to "0", and transistor Q1 169 is "open", meaning that output OUTS1 on the ASIC 161 is connected to output connector J3. Output 1-GP0 of microcontroller 159 exactly duplicates the signal from sensor S 167 (When sensor S 167 is disconnected, 1-GP0 shows a logical "1"; and when sensor S 167 is connected, 1-GP0 of microcontroller 159 shows a logical "0"). Alternatively, the sensor S 167 may be realized as an analog circuit, with appropriate modification of the microcontroller 159 to accommodate an analog device, for example, by including an analog-to-digital converter as is known in the art.

Therefore, the cigarette 165 works the same as usual, and the circuitry 149 does not affect the operation of the cigarette 165, since the input 6-GP3 of microcontroller 159 shows a logical "0", which indicates that the charger 150 is not connected.

### Scenario B: The charger 150 is connected.

As the charger 150 is connected, a logical "1" is instantly created on leg 6-GP3 of the microcontroller 159, which indicates that the charger 150 is connected.

At this state, a logical "1" is produced constantly at the output 1-GP0 of the microcontroller 159, meaning that the ASIC 161 no longer controls sensor S 167. The transistor Q1 169 is also closed in this state, meaning that there is no electrical connection between OUT1 of the ASIC 161 and the output connector J3.

When the sensor S 167 is not activated, meaning that the cigarette 165 is not being puffed, the voltage from the charger 150 is being directed via connectors J1, J2 to the input OUT1 of ASIC 161 and goes out through output OUT2 of ASIC 161, to the cell 163, resulting in the charging of the cell 163.

When the sensor S 167 is activated, meaning that the cigarette 165 is being puffed, the signal from the sensor S 167 arrives at the input 3-GP1 of the microcontroller 159, which then does the following:
At the input 4-GP2 a logical "0" is generated. Transistor Q1 169 opens, and the power travels from the charger 150 through the transistor Q1 169 and goes to the heating element of the cartridge (not shown) in the cigarette 165, and at the same time the power charges the cell 163 (if it is not fully charged).

In some cases, LED's (not shown) are included with the ASIC 161 or separate from the ASIC 161 and controlled by the microcontroller 159 in either case. These LED's inform the user of the status of the ASIC 161 and simulate smoking activity.

In another case, all the functionality of the circuitry 149 may be incorporated into the ASIC.

### Device 3.

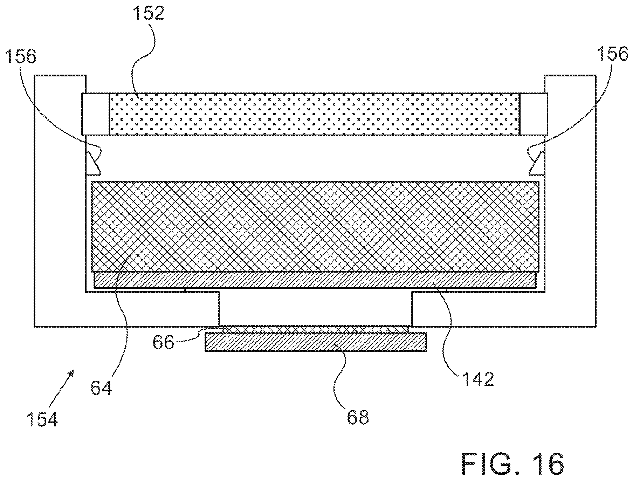

Reference is now made to Fig. 16, which is a side elevation of a cradle 154 for connection to a battery charging device. This version features a circular ring 152 that engages the tip of a cigarette, holding it in contact with the cradle 154. The ring 152 should be made of a rubbery material such as silicone, in order to facilitate holding a cigarette tip inside the cradle 154 by friction. Notches 156 retain magnet 64.

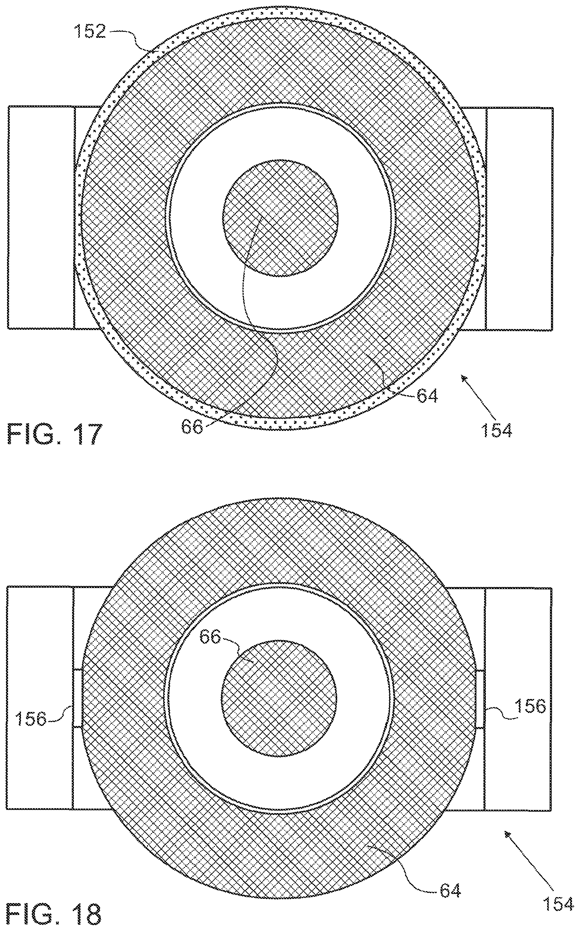

Reference is now made to Fig. 17, which is a top view of the cradle 154, showing the ring 152, and the magnets 64, 66 in place.

Reference is now made to Fig. 18, which is a sectional view through the cradle 154, illustrating the magnet 64 being held in place by the notches 156.

### Device 4

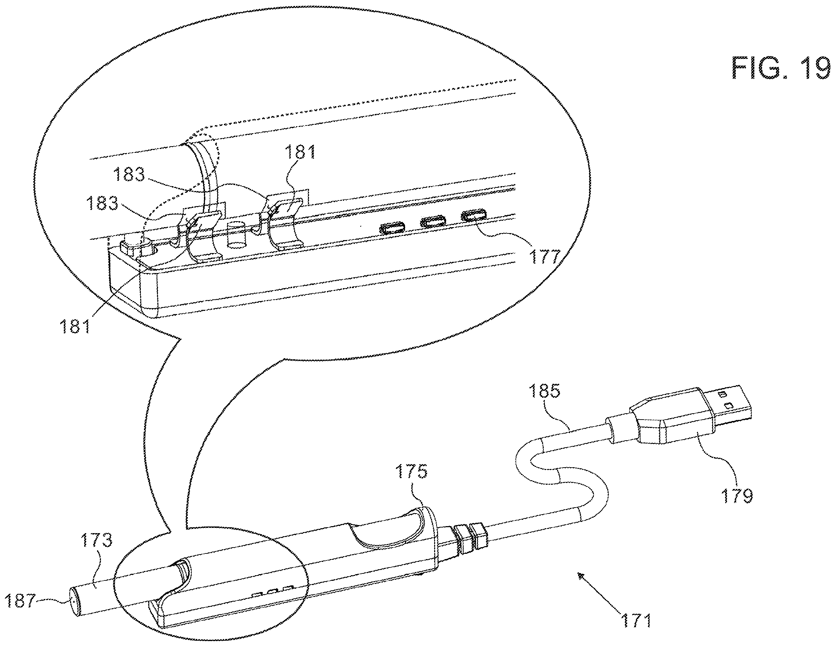

Reference is now made to Fig. 19, which is a partially cut-away view of an electronic cigarette charging system 171. This system, like the system shown in Fig. 14, uses a side-port on the electronic cigarette to connect to a cradle 175 housing battery charging circuitry 177. Use of the system 171 avoids any need for disassembly of an electronic cigarette 173. Indeed, the electronic cigarette 173 may be temporarily removed during charging, puffing resumed, and the electronic cigarette then replaced in the cradle 175 to continue charging the battery of the electronic cigarette.

A universal serial bus (USB) adaptor 179 connects a source of power (not shown), e.g., a laptop computer to the circuitry 177 via a cable 185. When the electronic cigarette 173 is inserted into the cradle 175, electrical contacts 181 on the cradle 175 mate with side port electrical contacts 183, which may be magnetic, or spring-loaded, and which are disposed along the barrel of the electronic cigarette 173, as best seen in Fig. 14

Similar to the tip-charging variation described above, the side-charging configuration can also allow for puffing while the cigarette is inside the cradle 175. In such case, the circuitry shown in Fig. 15 may be used.

### Device 5

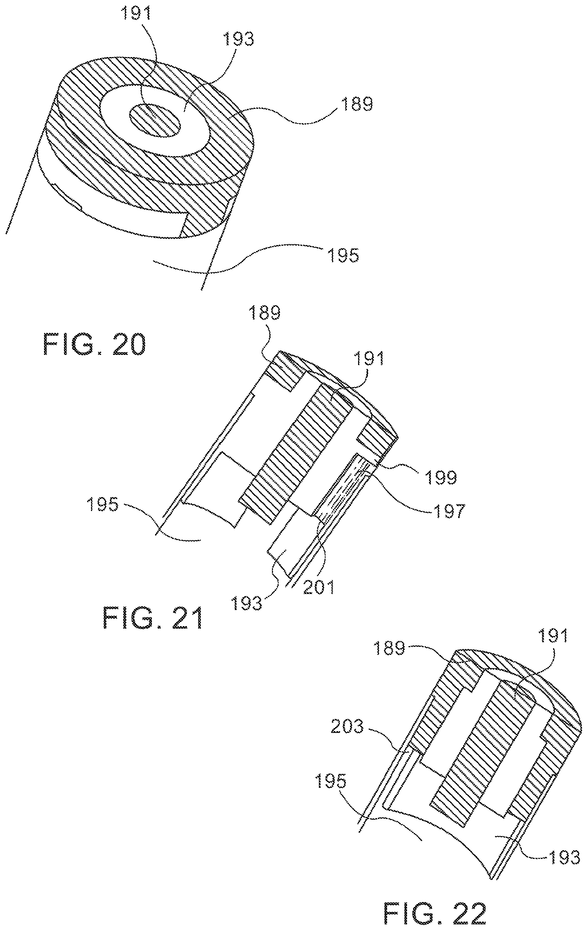

Reference is now made to Fig. 20, Fig. 21 and Fig. 22, which are, respectively, an elevation and two cutaway views of a tip of an electronic cigarette that is constructed and operative to engage a battery charger. An outer metallic ring or band 189 functions as one electrical contact with the battery charger. The band 189 is typically, but not necessarily, the positive contact. As seen in Fig. 22, it extends proximally, and is exposed to the interior of body 195 of the electronic cigarette at a point 203. A centrally located metallic plug 191 functions as the other electrical contact, typically the negative pole. The band 189 and the plug 191 may be magnetic, or constructed of a paramagnetic material. Wires (not shown), lead from the band 189 and the plug 191 to a battery within the body of the cigarette as discussed above, or to regulatory circuitry, e.g., circuitry 149 (Fig. 15).

A member 193 appears on Fig. 20 as a ring separating the band 189 from the plug 191.

The member 193 has a first function: it acts as an electrical insulator between the two contacts, band 189 and plug 191. It is formed of an electrically non-conductive material.

The member 193 has a second function: placing the interior of the body 195 into fluid communication with ambient air as seen in Fig. 21, so as to facilitate operation of a pressure sensor, e.g., sensor 24 (Fig. 1). The member 193 achieves this using a groove or channel 197 formed therein, which emerges onto the exterior of the tip at a point 199 and debouches into the interior of the body 195 at point 201.

The member 193 has a third function: simulating the glow of a conventional cigarette tip. To that end, the member 193 is typically composed of a plastic having an opacity and diffusing property so that light from a LED (not shown) within the body 195 diffuses when it passes through the member 193, so as to mask internal structures and internal shadows produced by the LED, such that their features cannot be perceived by an observer. This effect may be enhanced by including tiny crystals, e.g., glass crystals, within the plastic.

### Device 6

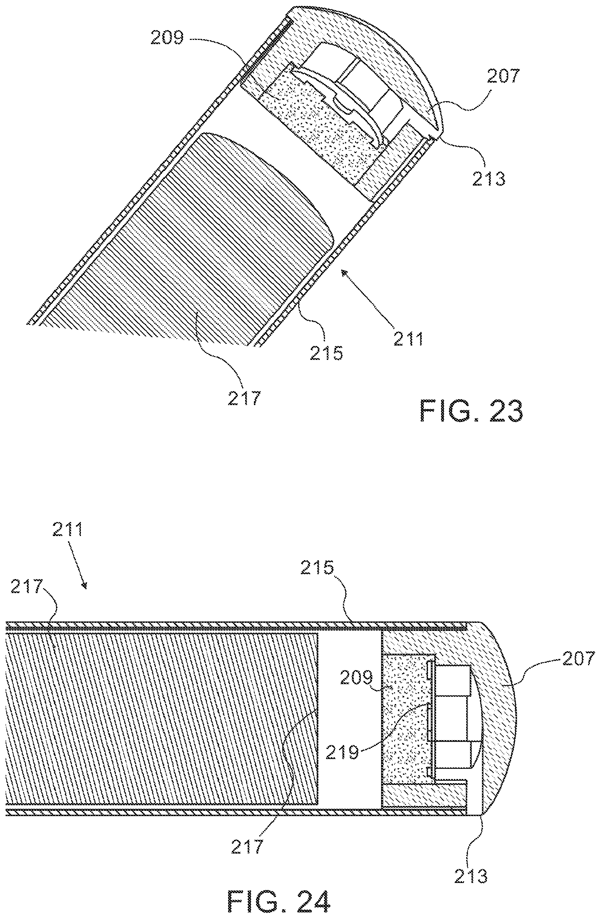

Reference is now made to Fig. 23, which is a cut-away view of a portion of a barrel 215 of an electronic cigarette 211 having a LED cap 207. This is a modification of the tip adaptor 34 shown in Figs. 2, 3 and 4, which accommodates a sensor in an internal chamber, and may include integrated circuitry. The LED cap 207 is mostly inset into the end of the barrel 215, and lacks the electrical contact mechanisms of the adaptor 34 for tip-charging the electronic cigarette. The LED cap 207 may be constructed of a plastic material as described for the member 193 (Fig. 20). A sensor unit 209 occupies the interior of the LED cap 207, and corresponds to the sensor 24 (Fig. 1), but instead of being inside a plastic housing of its own, the sensor unit 209 is now disposed inside the LED cap 207 in order to save space. Electrical circuitry associated with or incorporated in the sensor unit 209 may enable the atomizer and perform various additional functions as may be required for the operation of the electronic cigarette 211. The LED cap 207 has a perforation 213 for communication between the sensor unit 209 and the ambient atmosphere. A battery 217 powers the electronic cigarette 211 as described above.

Reference is now made to Fig. 24, which is a sectional view of the electronic cigarette 211 shown in Fig. 23, illustrating structural details of the sensor unit 209 and its relationship with the LED cap 207 and perforation 213.

It will be appreciated by persons skilled in the art that the present system is not limited to what has been particularly shown and described hereinabove. Rather, the system may include both combinations and sub-combinations of the various features described hereinabove, as well as variations and modifications thereof. The invention is defined by the appended claims.

## Claims

1. An electronic cigarette charging system, comprising:
a rechargeable electronic cigarette (10) having an end and first (74, 189) and second (82, 191) electrical contacts at the end, the second electrical contact arranged within the first electrical contact;
a cradle connectable to a battery charger, comprising:
a receiving element adapted to receive the end of the electronic cigarette (10), the receiving element having electrically conductive first and second contacts having respective first and second contact surfaces for contacting the first and second electrical contacts of the electronic cigarette (10), respectively to thereby establish an electrical connection with the battery charger, **characterized in that** the first electrical contact is a magnetic outer ring magnetically attractive to the corresponding contact of the receiving element.

2. The system according to claim 1, wherein the first (74, 189) and second (82, 191) electrical contacts of the electronic cigarette (10) are magnetically attractive to the first and second contact surfaces of the receiving element, respectively.

3. The system according to any of claims 1-2, wherein at least one of the first and second contact surfaces of the receiving element is a magnet.

4. The system according to any of claims 1-3, wherein at least one of the first (74, 189) and second (82, 191) electrical contacts of the electronic cigarette (10) is a magnet.

5. The system according to any of claims 1-4, further comprising an adaptor (34) having an orifice (46) formed therethrough and an inset for receiving a conductive flange (38), the orifice (46) being disposed such that an extension of the conductive flange (38) can be placed in contact with one of the first and second contacts of the receiving element, the adaptor (34) further comprising at least one side groove for receiving a contact pin (40) to contact another of the first and second contacts of the receiving element.

6. The system according to claim 5, wherein the adaptor (34) has vents (44) formed therein to permit ambient air to enter an interior of the electronic cigarette (10).

7. The system according to any of claims 1-4, wherein the end of the electronic cigarette comprises an adaptor having a sensor unit (24) housed in an interior chamber thereof, and having a perforation extending from the interior chamber to an exterior of the electronic cigarette to place the interior chamber in fluid communication with ambient atmosphere.

8. The system according to any of claims 1-4, wherein the end of the electronic cigarette (10) comprises an adaptor (70), the adaptor including:
a cap (72) having a lip (78) and a conductive flange (74) on the cap (72) for contacting one of the first and second contact surfaces of the receiving element (50), the conductive flange (74) being the first electrical contact of the electronic cigarette (10); and
a conductive coaxial member (82) having an insert (76) attached to the lip (78), the conductive coaxial member (82) being the second Electrical contact of the electronic cigarette (10), wherein the insert (76) has an orifice (80) formed therethrough for receiving an extension (88) of the conductive coaxial member (82), the orifice (80) being disposed such that the extension (88) of the conductive coaxial member (82) is placed in contact with another of the first and second contacts of the receiving element when the electronic cigarette (10) is in the cradle.

9. The system according to claim 8, wherein the adaptor has vents (92) formed therein to permit ambient air to enter an interior of the electronic cigarette (10).

10. The system according to any of claims 8-9, further comprising a light source within the electronic cigarette (10), wherein the insert (76) has a translucency and diffusing property suitable for preventing recognition of structures and shadows within the electronic cigarette (10) when the insert (76) is illuminated from within the electronic cigarette (10) by the light source.

## Patentansprüche

1. Ladesystem für elektronische Zigaretten, umfassend:
eine wiederaufladbare elektronische Zigarette (10), die ein Ende und einen ersten (74, 189) und einen zweiten (82, 191) elektrischen Kontakt an dem Ende hat, wobei der zweite elektronische Kontakt innerhalb des ersten elektrischen Kontakts angeordnet ist;
eine Ladestation, die mit einem Batterieladegerät verbindbar ist, umfassend:
ein Aufnahmeelement, das dafür eingerichtet ist, das Ende der elektronischen Zigarette (10) aufzunehmen, wobei das Aufnahmeelement einen elektrisch leitfähigen ersten und zweiten Kontakt mit einer jeweiligen ersten und zweiten Kontaktoberfläche zum Kontaktieren des ersten bzw. zweiten elektrischen Kontakts der elektronischen Zigarette (10) hat, um dadurch eine elektrische Verbindung mit dem Batterieladegerät herzustellen, **dadurch gekennzeichnet, dass** der erste elektrische Kontakt ein magnetischer Außenring ist, der für den entsprechenden Kontakt des Aufnahmeelements magnetisch anziehend ist.

2. System nach Anspruch 1, worin der erste (74, 189) und zweite (82, 191) elektrische Kontakt der elektronischen Zigarette (10) für die erste bzw. die zweite Kontaktoberfläche des Aufnahmeelements magnetisch anziehend sind.

3. System nach einem der Ansprüche 1 bis 2, worin mindestens eine der ersten und zweiten Kontaktoberfläche des Aufnahmeelements ein Magnet ist.

4. System nach einem der Ansprüche 1 bis 3, worin mindestens einer des ersten (74, 189) und zweiten (82, 191) elektrischen Kontakts der elektronischen Zigarette (10) ein Magnet ist.

5. System nach einem der Ansprüche 1 bis 4, ferner einen Adapter (34) umfassend, der eine dahindurch ausgebildete Öffnung (46) und einen Einsatz zum Aufnehmen eines leitfähigen Flanschs (38) hat, wobei die Öffnung (46) so angeordnet ist, dass ein Fortsatz des leitfähigen Flanschs (38) in Kontakt mit einem des ersten und zweiten Kontakts des Aufnahmeelements gebracht werden kann, wobei der Adapter (34) ferner mindestens eine seitliche Kerbe zum Aufnehmen eines Kontaktstifts (40) umfasst, um einen anderen des ersten und zweiten Kontakts des Aufnahmeelements zu kontaktieren.

6. System nach Anspruch 5, worin der Adapter (34) darin ausgebildete Lüftungsöffiiungen (44) hat, um zu ermöglichen, dass Umgebungsluft in ein Inneres der elektronischen Zigarette (10) eintritt.

7. System nach einem der Ansprüche 1 bis 4, worin das Ende der elektronischen Zigarette einen Adapter umfasst, der eine Sensoreinheit (24) hat, die in einer Innenkammer desselben untergebracht ist, und eine Perforation hat, die sich von der Innenkammer zu einer Außenseite der elektronischen Zigarette erstreckt, um die Innenkammer in Fluidkommunikation mit Umgebungsatmosphäre zu bringen.

8. System nach einem der Ansprüche 1 bis 4, worin das Ende der elektronischen Zigarette (10) einen Adapter (70) umfasst, wobei der Adapter aufweist:
eine Kappe (72) mit einer Lippe (78) und einem leitfähigen Flansch (74) auf der Kappe (72) zum Kontaktieren einer der ersten und zweiten Kontaktoberfläche des Aufnahmeelements (50), wobei der leitfähige Flansch (74) der erste elektrische Kontakt der elektronischen Zigarette (10) ist; und
ein leitfähiges koaxiales Bauteil (82) mit einem Einsatz (76), der an der Lippe (78) angebracht ist, wobei das leitfähige koaxiale Bauteil (82) der zweite elektrische Kontakt der elektronischen Zigarette (10) ist, worin der Einsatz (76) eine dahindurch ausgebildete Öffnung (80) zum Aufnehmen eines Fortsatzes (88) des leitfähigen koaxialen Bauteils (82) hat, wobei die Öffnung (80) so angeordnet ist, dass der Fortsatz (88) des leitfähigen koaxialen Bauteils (82) in Kontakt mit einem anderen des ersten und zweiten Kontakts des Aufnahmeelements gebracht wird, wenn die elektronische Zigarette (10) in der Ladestation ist.

9. System nach Anspruch 8, worin der Adapter darin ausgebildete Lüftungsöffnungen (92) hat, um zu ermöglichen, dass Umgebungsluft in ein Inneres der elektronischen Zigarette (10) eintritt.

10. System nach einem der Ansprüche 8 bis 9, ferner eine Lichtquelle innerhalb der elektronischen Zigarette (10) umfassend, worin der Einsatz (76) eine Lichtdurchlässigkeits- und Streueigenschaft hat, die zum Verhindern der Erkennung von Strukturen und Schatten innerhalb der elektronischen Zigarette (10) geeignet ist, wenn der Einsatz (76) durch die Lichtquelle aus dem Inneren der elektronischen Zigarette (10) beleuchtet wird.

## Revendications

1. Système de charge pour cigarette électronique, comprenant :
une cigarette électronique rechargeable (10) ayant une extrémité et des premier (74, 189) et deuxième (82, 191) contacts électriques au niveau de l'extrémité, le deuxième contact électrique étant agencé à l'intérieur du premier contact électrique ;
un support qui peut être connecté à un chargeur de batterie, comprenant :
un élément de réception adapté pour recevoir l'extrémité de la cigarette électronique (10), l'élément de réception ayant des premier et deuxième contacts électriquement conducteurs ayant des première et deuxième surfaces de contact respectives destinées à entrer en contact avec les premier et deuxième contacts électriques de la cigarette électronique (10), respectivement pour ainsi établir une connexion électrique avec le chargeur de batterie, **caractérisé en ce que** le premier contact électrique est une bague externe magnétique attirant le contact correspondant de l'élément de réception.

2. Système selon la revendication 1, dans lequel les premier (74, 189) et deuxième (82, 191) contacts électriques de la cigarette électronique (10) attirent magnétiquement les première et deuxième surfaces de contact de l'élément de réception, respectivement.

3. Système selon l'une quelconque des revendications 1-2, dans lequel au moins une des première et deuxième surfaces de contact de l'élément de réception est un aimant.

4. Système selon l'une quelconque des revendications 1-3, dans lequel au moins un des premiers (74, 189) et deuxièmes (82, 191) contacts électriques de la cigarette électronique (10) est un aimant.

5. Système selon l'une quelconque des revendications 1-4, comprenant en outre un adaptateur (34) ayant un orifice (46) formé à travers celui-ci et un élément inséré destiné à recevoir une bride conductrice (38), l'orifice (46) étant disposé de telle sorte qu'une extension de la bride conductrice (38) peut être mise en contact avec l'un des premier et deuxième contacts de l'élément de réception, l'adaptateur (34) comprenant en outre au moins une rainure latérale destinée à recevoir une broche de contact (40) pour entrer en contact avec un autre des premier et deuxième contacts de l'élément de réception.

6. Système selon la revendication 5, dans lequel l'adaptateur (34) a des aérations (44) formées dans celui-ci pour permettre à l'air ambiant d'entrer dans un intérieur de la cigarette électronique (10).

7. Système selon l'une quelconque des revendications 1-4, dans lequel l'extrémité de la cigarette électronique comprend un adaptateur ayant une unité de détection (24) logée dans une chambre intérieure de celui-ci, et ayant une perforation s'étendant de la chambre intérieure à un extérieur de la cigarette électronique pour mettre la chambre intérieure en communication fluidique avec l'atmosphère ambiante.

8. Système selon l'une quelconque des revendications 1-4, dans lequel l'extrémité de la cigarette électronique (10) comprend un adaptateur (70), l'adaptateur comportant :
un coiffe (72) ayant une lèvre (78) et une bride conductrice (74) sur la coiffe (72) destinée à entrer en contact avec l'une des première et deuxième surfaces de contact de l'élément de réception (50), la bride conductrice (74) étant le premier contact électrique de la cigarette électronique (10) ; et
un élément coaxial conducteur (82) ayant un insert (76) rattaché à la lèvre (78), l'élément coaxial conducteur (82) étant le deuxième contact électrique de la cigarette électronique (10), dans lequel l'insert (76) a un orifice (80) formé à travers celui-ci destiné à recevoir une extension (88) de l'élément coaxial conducteur (82), l'orifice (80) étant disposé de telle sorte que l'extension (88) de l'élément coaxial conducteur (82) est mise en contact avec un autre des premier et deuxième contacts de l'élément de réception lorsque la cigarette électronique (10) est dans le support.

9. Système selon la revendication 8, dans lequel l'adaptateur a des aérations (92) formées dans celui-ci pour permettre à l'air ambiant d'entrer dans un intérieur de la cigarette électronique (10).

10. Système selon l'une quelconque des revendications 8-9, comprenant en outre une source de lumière à l'intérieur de la cigarette électronique (10), dans lequel l'insert (76) a une propriété de translucidité et de diffusion adéquate pour empêcher la reconnaissance de structures et d'ombres à l'intérieur de la cigarette électronique (10) lorsque l'insert (76) est éclairé depuis l'intérieur de la cigarette électronique (10) par la source de lumière.
